# EUROPÄISCHE PATENTSCHRIFT

(11) **EP 4 539 903 B1**
(45) Veröffentlichungstag und Bekanntmachung des Hinweises auf die Patenterteilung: **04.11.2026**
(21) Anmeldenummer: 23733270.5
(22) Anmeldetag: 14.06.2023
(51) Int. Cl.: A61M 1/16

(54) **VORRICHTUNG ZUR HERSTELLUNG VON DIALYSIERFLÜSSIGKEIT**
DEVICE FOR THE PRODUCTION OF DIALYSIS FLUID
DISPOSITIF POUR LA PRODUCTION DE LIQUIDE DE DIALYSE

(30) Priorität: 14.06.2022 DE 102022114956
(43) Veröffentlichungstag der Anmeldung: 23.04.2025
(73) Patentinhaber: B. Braun Avitum AG, 34212 Melsungen (DE)
(72) Erfinder: NIEMETZ, Günter, 34212 Melsungen (DE); KUCHENBÄCKER, Ralf, 34225 Baunatal (DE)
(74) Vertreter: Winter, Brandl - Partnerschaft mbB
(86) Internationale Anmeldenummer: PCT/EP2023/065915
(87) Internationale Veröffentlichungsnummer: WO 2023/242242

(56) Entgegenhaltungen:
- US-A1- 2015 041 377

## Beschreibung

### Technisches Gebiet

Die vorliegende Offenbarung betrifft eine Vorrichtung für eine oder einer extrakorporalen Blutbehandlungsmaschine zur Herstellung und/oder Aufbereitung einer Dialysierflüssigkeit bei (gleichzeitiger) Reduzierung einer Leitfähigkeitsschwankung in der Ionen-versetzten osmotischen Flüssigkeit (Osmosewasser). Ferner betrifft die vorliegende Offenbarung ein Ansteuerungs-/Regelungsverfahren einer Dosierpumpe zur Herstellung und/ oder Aufbereitung von Dialysierflüssigkeit.

In anderen Worten betrifft die vorliegende Offenbarung eine Vorrichtung und ein Verfahren zur Herstellung von Dialysierflüssigkeit, bei welchem saure und/oder basische Komponenten einem Osmose-Wasser in möglichst präziser Weise hinzugefügt werden.

### Hintergrund der Erfindung

Bei der extrakorporalen Blutbehandlung, zum Beispiel Hämodialyse, Hämofiltration, Hämodiafiltration usw., wird mittels einer Dialysemaschine die Dialysierflüssigkeit aus den Grundbestandteilen osmotisches Wasser, basisches Fluid und saures Fluid oder Acetat hergestellt. Die Dialysierflüssigkeit wird während der Dialysebehandlung eines Patienten durch die dialysierflüssigkeitsseitige Kammer eines Dialysators geleitet und über dessen semipermeable Membran werden durch Diffusion (Hämodialyse) oder Diffusion in Kombination mit Konvektion (Hämofiltration oder Hämodiafiltration) urämische Toxine und Wasser aus dem Blut, das durch die blutseitige Kammer des Dialysators geleitet wird, aufgenommen.

Während der extrakorporalen Blutbehandlung wird das zu behandelnde Blut des Patienten in dem Dialysator durch semipermeable Hohlfasermembrane geleitet, die von der Dialysierflüssigkeit umspült werden. Bei einer basischen (ersten) Komponente der Dialysierflüssigkeit handelt es sich für gewöhnlich um ein Substrat mit Natriumhydrogencarbonat, bei einer sauren (zweiten) Komponente handelt es sich gewöhnlicher Weise um eine Lösung mit Natriumchlorid, Kaliumchlorid, Magnesiumchlorid, Calciumchlorid, Glucose, Essigsäure und/oder Zitronensäure oder es wird nur eine Komponente verwendet, die dann gewöhnlicher Weise aus Acetat besteht.

Zur Herstellung der Dialysierflüssigkeit mittels eines Regelkreises werden Dosierpumpen und Leitfähigkeitssonden eingesetzt. Dabei misst eine Sonde die Leitfähigkeit des osmotischen Wassers/ dem Grundbestandteil der Dialysierflüssigkeit nach der Zugabe der bzw. mit der eingemischten basischen Komponente mittels einer Dosierpumpe und über eine Rechnereinheit wird der Istwert der Leitfähigkeit des mit der basischen Komponente versetzten Wassers gegen einen Sollwert verglichen und über die Drehzahl der Dosierpumpe, wenn nötig nachgeregelt. Nach der Zugabe der sauren Komponente mittels einer weiteren Dosierpumpe misst eine weitere Sonde die Leitfähigkeit der gesamten Dialysierflüssigkeit und über eine Rechnereinheit wird der Istwert der Leitfähigkeit der gesamten Dialysierflüssigkeit gegen einen weiteren Sollwert verglichen und über die Drehzahl dieser weiteren Dosierpumpe, wenn nötig nachgeregelt.

Anders ausgedrückt, wird das osmotische Wasser mit einer basischen und ggf. einer sauren Komponente versetzt. Für die basische und die saure Komponente sind separate Behälter vorgesehen, in denen entsprechende Lösungen mit bestimmter Stoffkonzentration enthalten sind. Ferner sind Dosierpumpen vorgesehen, die die entsprechenden Lösungen aus den Behältern pumpen und dem osmotischen Wasser zusetzen. Dabei ist es von erheblicher Bedeutung, dass das Mischungsverhältnis stimmt.

Um dies zu gewährleisten, werden die elektrische Leitfähigkeits-Messsonden verwendet, welche die elektrische Leitfähigkeit des Osmose-Wassers vor/nach jeder Zugabe der einzelnen Lösungen messen, wobei ein Rechner aus den Leitfähigkeitsänderungen Rückschlüsse auf die Konzentration der jeweils gerade zugesetzten Stoffe ziehen kann, welche in den Lösungen enthalten sind. Das heißt der Rechner ist auf Basis der Leitfähigkeits-Messergebnisse in der Lage, die Dosierung der einzelnen Lösungen, in denen Stoffe mit einer bestimmten Konzentration gelöst sind, so zu regeln, dass die Konzentration an Stoffen im osmotischen Wasser jeweils einen bestimmten Wert annimmt.

### Stand der Technik

Dosierregelungen regeln sowohl die Störeinflüsse, zum Beispiel Flussschwankungen des Osmose-Wassers oder Luftblasen in den Konzentrat-Quellen, sowie die Fördercharakteristiken der Dosierpumpen über die gesamte Regelstrecke. Da die Dosierpumpen, wie zum Beispiel Drehschieberkolbenpumpen oder Membranpumpen und dergleichen keine kontinuierliche Fördercharakteristik haben, führt dies zu ständigen Leitfähigkeitsschwankungen, die die Regelung nie vollkommen ausregeln kann. Die Dosierpumpen weisen vielmehr eine Ansaugphase und eine Auslassphase auf. In der Ansaugphase wird ein Fluid von der Dosierpumpe bzw. einem Kolben der Dosierpumpe angesaugt und in der Auslassphase wird das Fluid von der Dosierpumpe bzw. dem Kolben der Dosierpumpe gefördert/ausgelassen. D.h. während der Ansaugphase kann die Dosierpumpe nicht fördern. Daher hat das von der Dosierpumpe geförderte Volumen einen sinusförmigen Verlauf mit einem Ansaughub und einem Auslasshub.

Zur Reduzierung der Störeinflüsse durch die diskontinuierliche Fördercharakteristik der Dosierpumpen werden oftmals zusätzliche Misch- oder Dämpferkammern verbaut, aus denen dann kontinuierlich Flüssigkeit mit im wesentlichen konstanter Mischung entnommen werden kann. Jedoch führen diese zu Mehrkosten und erhöhen zusätzlich das im System enthaltene Flüssigkeitsvolumen.

Die verwendete Schrittmotoransteuerung besteht dabei aus einem Treiber, der die Phasenwicklungen des Schrittmotors wechselweise bestromt, so dass der Schrittmotor bei jedem Ansteuerimpuls eine Rotationsbewegung um einen Auflösungsschritt macht. Die Auflösungsschritte sind dabei von der mechanischen Konstruktion der Wicklungen des Schrittmotors vorgegeben (zum Beispiel 200 Schritte pro Umdrehung, das heißt 1,8° Schrittwinkel pro Ansteuerimpuls).

Aus der DE 10 2018 010 174 A1 ist eine Vorrichtung zur Herstellung einer Dialysierflüssigkeit bekannt, wobei die Vorrichtung eine Fluidleitung zur Führung einer Dialysierflüssigkeit sowie Mittel aufweist, die ausgebildet sind, eine Dialysierflüssigkeit in der Fluidleitung diskontinuierlich zu fördern, wobei die Vorrichtung einen Leitfähigkeitssensor aufweist, der angeordnet ist, die Leitfähigkeit der Dialysierflüssigkeit zu messen, wobei die Vorrichtung eine stromaufwärts des Leitfähigkeitssensors in die Dialysierflüssigkeitsleitung an einer Zugabestelle mündende Konzentratleitung mit einer Konzentratpumpe aufweist sowie einen Konzentratbehälter, aus dem die Konzentratpumpe Konzentrat in die Konzentratleitung und aus dieser in die Dialysierflüssigkeitsleitung fördert. Demnach ist eine Reduzierung der Leitfähigkeitsschwankungen mittels einer Dosierpumpe und nachfolgender Leitfähigkeitssonde bekannt.

Die DE 10 2010 015 293 A1 beschreibt eine hydraulische Servolenkung mit einer Pumpeinheit zur Erzeugung von Volumenstrom- und Druckpulsationen. Die Pumpeinheit ist über eine Eingangsleitung mit einem Hydraulikmittelbehälter und über eine Ausgangsleitung mit einem Lenkventil verbunden. In der Ausgangsleitung ist ein einstellbares Stromventil angeordnet und zur Steuerung des Stromventils ist eine Steuereinrichtung vorgesehen.

Die DE 10 2014 109 369 A1 offenbart eine Dialysemaschine, die eine Aufbereitungsanlage mit einer Hauptleitung für Dialysierflüssigkeit aufweist, an der mehrere Zuführleitungen für jeweilige Konzentrate münden. Nach jeder Mündung sind eine jeweilige Mischkammer und danach eine jeweilige Leitfähigkeitssonde angeordnet. Nach der letzten Leitfähigkeitssonde ist eine gemeinsame Pumpe angeordnet. Der Unterdruck in der Hauptleitung wird über ein Druckregelventil eingestellt, das vor der ersten Mündung in der Hauptleitung angeordnet ist. Weiterhin sind zur Dosierung der verschiedenen Konzentrate in jeder Zuführleitung ein Proportionalventil oder ein digitales Schaltventil vorgesehen. Das Druckregelventil und die Proportionalventile bzw. Schaltventile werden elektromagnetisch von einer zentralen elektronischen Steuereinheit gesteuert.

EP 3 843 806 A1 betrifft eine Vorrichtung und ein Verfahren zur Herstellung einer Dialysierflüssigkeit zur Verwendung bei einer extrakorporalen Blutbehandlung, wobei die Vorrichtung eine Hauptleitung zur Zufuhr von Wasser, vorzugsweise Osmose- oder Reinstwasser, umfasst, in dessen Verlauf eine Säure und eine Grundflüssigkeit jeweils in einer bestimmten Dosierung eingebracht werden, wobei die Dosierung durch eine Steuer- und Regeleinheit in Abhängigkeit von mindestens einem chemischen und/oder physikalischen Parameter, vorzugsweise der Leitfähigkeit, des Wasser/Flüssigkeits-Gemisches eingestellt wird, und der mindestens eine chemische und/oder physikalische Parameter von einer Messeinrichtung, insbesondere einer Leitfähigkeitsmesssonde, erfasst wird, wobei eine erste, vorzugsweise eine einzige Messeinrichtung an einem jeweils stromabwärts gelegenen Abschnitt der Hauptleitung der Einleitungsstelle für das saure Fluid und stromabwärts der Einleitungsstelle für das basische Fluid angeordnet ist, und die Steuer- und Regeleinheit zumindest zeitweise die Einleitung des sauren Fluids und das basische Fluid derart steuert, dass nur eines der sauren und basischen Fluide über einen vorbestimmten Zeitraum oder ein vorbestimmtes Intervall in die Hauptleitung eingeführt wird.

Aus der US 6 457 944 B1 ist eine Dosierpumpe bekannt, die derart gesteuert ist, dass eine Auslassphase der Dosierpumpe länger als eine Ansaugphase ist. Die Dosierpumpe wird von einem Asynchronmotor angetrieben, der während der Auslassphase mit einem Strom mit einer geringeren Frequenz beaufschlagt wird als in der Ansaugphase. Dadurch ist die Drehgeschwindigkeit des Asynchronmotors in der Auslassphase geringer als in der Ansaugphase. Dadurch kann eine möglichst gleichmäßige Fluidabgabe durch die Dosierpumpe erzielt werden.

Um das Fluid durch die Dosierpumpe möglichst gleichmäßig abzugeben, muss die Ansteuerung der Dosierpumpe mit der Ansaug- und Auslassphase der Dosierpumpe synchronisiert werden. Dazu muss erfasst werden, in welcher Position der elektrische Antrieb der Dosierpumpe ist. Bei Asynchronmotoren wie in der US 6 457 944 B1 wird dafür die Position des Motors durch einen Positionsencoder oder Drehgeber erfasst. Der Start der Ansaug- und/oder Auslassphase der Dosierpumpe wird zusätzlich durch Sensoren an der Dosierpumpe erfasst. Die durch den Positionsencoder erfasste Position des Asynchronmotors wird dann mit dem Start der Ansaug- und/oder Auslassphase der Dosierpumpe synchronisiert, um die möglichst gleichmäßige Fluidabgabe zu ermöglichen. Die Verwendung eines Positionsencoders verursacht aber Kosten und resultiert in einem höheren Steuer- oder Regelungsaufwand für den Motor der Dosierpumpe.

Die US 2015 / 041 377 A1 offenbart eine Vorrichtung einer Dialysemaschine zur Herstellung und/oder Aufbereitung einer Dialysierflüssigkeit, wobei die Vorrichtung einen Leitfähigkeitssensor und eine Dosierpumpe aufweist, und die Dolsierpumpe von einem Schrittmotor angetrieben wird.

### Beschreibung der vorliegenden Offenbarung

Der vorliegenden Offenbarung liegt die Aufgabe zugrunde, den vorstehenden Regel- oder Steuerkreis dahingehend zu optimieren, dass mit vergleichsweise geringem regelungs- oder steuerungstechnischen Aufwand eine zeitlich stabile elektrische Leitfähigkeit der Dialyselösung am bzw. im Dialysator erzeugbar ist/ erzeugt werden kann und damit ein konstantes Mischungsverhältnis in der Dialysierflüssigkeitsströmung gewährleistet ist.

Diese Aufgabe wird gelöst durch eine Vorrichtung für eine oder einer extrakorporalen Blutbehandlungsmaschine zur Herstellung und/oder Aufbereitung einer Dialysierflüssigkeit mit den Merkmalen des Anspruchs 1, ein Ansteuerung- oder Regelungssverfahren einer Dosierpumpe zur Herstellung und/ oder Aufbereitung von Dialysierflüssigkeit mit den Merkmalen des Anspruchs 9, ein computerlesbares Speichermedium mit den Merkmalen des Anspruchs 13 und ein Computerprogramm den Merkmalen des Anspruchs 14.

Die vorliegende Offenbarung betrifft demzufolge eine Vorrichtung / Regelungs- oder Steuerungsvorrichtung für eine oder einer extrakorporalen Blutbehandlungsmaschine zur Herstellung und/oder Aufbereitung einer Dialysierflüssigkeit. Die Vorrichtung weist eine Fluidhauptleitung zur Einleitung von osmotischem Wasser, mindestens eine Fluidnebenleitung, welche an die Fluidhauptleitung für die Zufuhr eines Additivs in das osmotische Wasser angeschlossen ist und eine in der Fluidnebenleitung angeordnete Dosierpumpe auf, die in einer Ansaugphase/einem Ansaughub das Additiv aus einem Vorrat ansaugt und in einer/einem seriell sich anschließenden Auslassphase/Auslasshub das Additiv in das osmotische Wasser innerhalb der Fluidhauptleitung ausdrückt. Ferner weist die Vorrichtung einen elektrischen Antrieb/eine Antriebseinheit zum Antreiben/Betreiben der Dosierpumpe und eine Steuer- oder Regelungseinheit zur Steuerung oder Regelung des elektrischen Antriebs auf, die den elektrischen Antrieb derart steuert oder regelt, dass die Ansaugphase der Dosierpumpe kürzer ist als die Auslassphase der Dosierpumpe. Der elektrische Antrieb ist ein Schrittmotor zum Betreiben der Dosierpumpe und die Dosierpumpe weist einen magnetischen Sensor auf, über den ein Positionssignal eines Kolbens der Dosierpumpe erfassbar ist. Die Steuer- oder Regelungseinheit synchronisiert einen Start der Ansaugphase und/oder der Auslassphase mit dem erfassten Positionssignal.

Anders ausgedrückt kann die Steuer- oder Regelungseinheit dafür ausgebildet und vorbereitet sein, den Schrittmotor synchron mit einem Start der Ansaugphase und/oder der Auslassphase durch das (durch den magnetischen Sensor) erfasste Positionssignal anzusteuern. Synchron ansteuern kann in diesem Zusammenhang dahingehend verstanden werden, dass die Steuer- oder Regelungseinheit das (Ansteuer-)Signal an den Schrittmotor, vorzugsweise die Frequenz des Stromes, dann ändert, wenn die Ansaugphase und/oder die Auslassphase der Dosierpumpe startet. D.h. die Steuer- oder Regelungseinheit variiert den Strom an den Schrittmotor bei einem Phasenwechsel der Dosierpumpe. Dadurch kann eine wirksame Glättung der Fluidabgabe der Dosierpumpe sichergestellt werden. Konkreter kann die Steuer- oder Regelungseinheit den Schrittmotor genau dann ansteuern also mit einem Strom beaufschlagen, wenn die Dosierpumpe die Ansaugphase und/oder die Auslassphase startet oder beendet. Die Steuer- oder Regelungseinheit kann also durch das erfasste Positionssignal erkennen, wann die Ansaugphase und/oder der Auslassphase startet (oder endet) und Schrittmotor basierend auf dem Positionssignal bzw. dem Start der Ansaugphase und/oder der Auslassphase ansteuern.

Der Schrittmotor als elektrischer Antrieb kann sich insbesondere dadurch auszeichnen, dass eine (physikalische) Position des Schrittmotors bekannt ist, da die Position des Schrittmotors aus der Anzahl der getätigten Schritte berechenbar sein kann. Schrittmotoren können auch einen drehzahlkonstanten oder exakt einstellbaren Betrieb ohne zusätzliche Sensoren und/oder Drehzahlregler ermöglichen.

Der magnetische Sensor kann insbesondere an der Dosierpumpe angebracht sein und erfasst das Positionssignal der Dosierpumpe.

In anderen Worten stellt die Vorrichtung Dialysierflüssigkeit aus den Grundbestandteilen (osmotisches) Wasser, basisches Fluid und ggf. saures Fluid oder nur aus Acetat her. Hierfür werden mittels eines Regel- oder Steuerkreises zumindest die Dosierpumpe und zumindest eine elektrische Leitfähigkeitssonde eingesetzt. Die Dosierpumpe fördert das zumindest eine Additiv in das osmotische Wasser in der Fluidhauptleitung und wird hierfür über den Schrittmotor angetrieben. Der Schrittmotor als elektrischer Antrieb wird von der Steuer- oder Regelungseinheit bzw. einer Ansteuerungseinheit der Steuer- oder Regelungseinheit angesteuert. Die Steuer- oder Regelungseinheit steuert / regelt den Schrittmotor der Dosierpumpe dabei derart, dass eine gewünschte Konzentration des Additivs in der Fluidhauptleitung stromab zur Einmündungsstelle der Fluidnebenleitung eingestellt wird. Die Konzentration des Additivs wird vorzugsweise von der elektrischen Leitfähigkeitssonde über eine elektrische Leitfähigkeit des (gemischten) Fluides erfasst und weiter vorzugsweise als Rückführung des Regelkreises an die Steuer- oder Regelungseinheit zurückgeführt. Dabei hat die Dosierpumpe die Ansaugphase und die Auslassphase, wodurch eine diskontinuierliche (sinusförmige) Fördercharakteristik der Dosierpumpe bedingt ist. Der Schrittmotor wird derart von der Steuer- oder Regelungseinheit angesteuert oder geregelt, dass die Dauer von einem Start der Ansaugphase bis zu einem Ende der Ansaugphase und damit zu einem Start der Auslassphase kleiner ist als die Dauer von dem Start der Auslassphase zu einem Ende der Auslassphase. Konkreter ist eine Dauer des Ansaughubs kleiner als eine Dauer des Auslasshubs. Das angesaugte Fluidvolumen während der Ansaugphase ist aber gleich einem geförderten / ausgelassenen Fluidvolumen der Dosierpumpe während der Auslassphase.

Vorzugsweise ist die Dosierpumpe eine Kolbenpumpe oder eine Membranpumpe und weist daher den einen Ansaughub und den einen Auslasshub auf.

Es kann sich bei der mindestens einen Dosierpumpe auch um eine erste Dosierpumpe und eine zweite Dosierpumpe handeln, wobei die erste Dosierpumpe ein erstes Additiv und die zweite Dosierpumpe ein zweites Additiv (unabhängig zur ersten Dosierpumpe) fördert.

Der Schrittmotor kann direkt von der Steuer- oder Regelungseinheit angesteuert oder geregelt werden. Der Schrittmotor kann aber auch von einer Ansteuereinheit angesteuert werden, die optional Teil der Steuer- oder Regelungseinheit sein kann.

Der Kern der Offenbarung besteht also darin, dass ein Schrittmotor einer Dosierpumpe derart angesteuert wird, dass eine Ansaugphase der Dosierpumpe kürzer als eine Auslassphase der Dosierpumpe ist, um ein schwankendes Fördervolumen der Dosierpumpe auszugleichen. Durch die Verwendung des Schrittmotors als Antrieb der Dosierpumpe kann die Position des Schrittmotors und damit eines Aktors der Dosierpumpe aus der Anzahl der getätigten Schritte bestimmt werden und der Schrittmotor somit synchron mit dem Start der Ansaug- oder Auslassphase angesteuert werden.

Als die (physikalische) Position des Schrittmotors kann in diesem Kontext insbesondere eine Umdrehung des Schrittmotors bzw. eine Anzahl von (getätigten) Schritten des Schrittmotors verstanden werden. Dabei kann die Position des Schrittmotors insbesondere mit einem vorgegebenen Drehwinkel eines Elektromotors verglichen werden. Im Gegensatz zum Drehwinkel des Elektromotors kann die Position des Schrittmotors aber ohne Sensorik erfasst werden, indem die Anzahl der (getätigten) Schritte des Schrittmotors erfasst wird.

Durch die Verkürzung der Ansaugphase im Vergleich zu der Auslassphase wird die Dauer des Ansaughubs der Dosierpumpe gegenüber der Dauer des Auslasshubs verkürzt. D.h. die Dosierpumpe gibt über einen längeren Zeitraum Additiv aus, als durch die Dosierpumpe angesaugt wird. Dadurch wird das Additiv nicht auf einmal, sondern über einen bestimmten Zeitraum kontinuierlich in das osmotische Wasser abgegeben. Durch die Verkürzung der Ansaugphase wird die Zeit verkürzt, in der kein Additiv dem osmotischen Wasser zugeführt wird. Die Leitfähigkeitssonde, die die Konzentration des Additivs in dem osmotischen Wasser (in der Fluidhauptleitung) nach der Dosierpumpe erfasst, detektiert die Zugabe des Additiv somit über einen längeren Zeitraum. Das reduziert einen Einfluss der erfassten Leitfähigkeitsschwankungen auf die Steuerung oder Regelung der Dosierpumpe, die auf der von der Leitfähigkeitssonde erfassten Leitfähigkeit basiert.

Insgesamt kann somit mit einfachen Mitteln eine (annähernd) kontinuierliche Fördercharakteristik der Dosierpumpe ermöglicht werden. Damit wird der Einfluss der unregelmäßigen Fördercharakteristik auf den Regelkreis der Dosierpumpe vermindert. Insbesondere kann der Einsatz von zusätzlichen Mischkammern vermieden werden, die bei bekannten Dosierpumpen verwendet werden, um die Störeinflüsse durch eine diskontinuierliche Fördercharakteristik bekannter Dosierpumpen auszugleichen.

Durch die Verwendung des Schrittmotors als elektrischen Antrieb der Dosierpumpe kann auf einen Drehgeber oder Positionsencoder für den elektrischen Antrieb verzichtet werden. Bei einem Schrittmotor kann die Position des Schrittmotors ohne zusätzliche Sensorik erfasst werden und der Schrittmotor kann somit kostengünstig und einfach mit der Auslass- und/oder Ansaugphase synchronisiert werden. Damit kann eine konstantere Fluidabgabe gewährleistet werden.

Weitere vorteilhafte Weiterbildungen gemäß den Unteransprüchen werden nachfolgend detailliert beschrieben.

Es ist bevorzugt, wenn der magnetische Sensor ein Reedsensor oder ein Hallsensor ist. Der magnetische Sensor kann insbesondere an der Dosierpumpe angebracht sein, um das Positionssignal der Dosierpumpe und insbesondere den Start der Ansaug- und/oder Auslassphase zu bestimmen. Wenn es sich bei der Dosierpumpe um eine Drehschieberkolbenpumpe handelt, kann eine Position des Kolbens der Dosierpumpe durch den magnetischen Sensor erfasst werden. Somit kann die Position (des Kolbens) der Dosierpumpe kostengünstig und einfach ermittelt werden.

Bei einer Drehschieberkolbenpumpe kann sich der Kolben zwischen zwei Totpunkten eines Kolbenhubs bewegen. Vorzugsweise kann der magnetische Sensor das Positionssignal der Dosierpumpe an den jeweiligen Totpunkten erfassen. D.h. der magnetische Sensor kann der Steuer- oder Regelungseinheit rückmelden, wann der Kolben in einem Totpunkt ist. Vorzugsweise entspricht eine Positionierung des Kolbens im Totpunkt dem Start der Ansaugphase und/ oder der Auslassphase. Somit kann durch den magnetischen Sensor der Start der Ansaugphase und/ oder der Auslassphase ermittelt werden.

Es kann vorteilhaft sein, wenn der magnetische Sensor als eine Positionsbestimmungseinheit dazu vorgesehen ist, eine Synchronisation der physikalischen Position mit einem Start der Einlass-/Ansaugphase und/ oder der Austrags-/Auslassphase durchzuführen. Dazu kann vorzugsweise der Schrittmotor mit dem Start der Einlass-/Ansaugphase und/ oder der Austrags-/Auslassphase synchronisiert werden. Der Start der Einlass-/Ansaugphase und/ oder der Austrags-/Auslassphase kann dabei durch den magnetischen Sensor an der Dosierpumpe erfasst werden. Daraufhin kann der Schrittmotor basierend auf dem erfassten Signal synchronisiert werden.

Bei dem Einsatz eines Schrittmotors als elektrischen Antrieb kann also eine Synchronisation der physikalischen Position mit einem magnetischen Sensor ausreichend sein (im Gegensatz zu einer Synchronisation über einen Drehgeber), da ein Schrittmotor positionsgenau gestellt werden kann.

Die aktuelle Position des Schrittmotors kann sich durch Zählen der getätigten Schrittimpulse nach dem letzten Positionssignal ergeben. Nach einer erstmaligen Synchronisation der Position berechnet die Ansteuerungseinheit die zeitliche Auflösung der Ansteuerimpulse für den Schrittmotor zum Erzielen einer bestmöglichen Kompensation der Ansaug-/Einlass- und Auslass-/Austragsphase und erzeugt diese Impulse in einem erforderlichen Zeitraster.

Für die Umsetzung der Ansteuerung mittels der Ansteuerungseinheit kann es also vorteilhaft sein, die physikalische Position des Schrittmotors zu erkennen. Dadurch kann die physikalische Position des Schrittmotors mit dem Start der Ansaugphase und/oder der Auslassphase synchronisiert werden. Bei der Verwendung des Schrittmotors als elektrischem Antrieb kann die physikalische Position des Schrittmotors aus den getätigten Schritten berechnet werden. Alternativ kann die Dosierpumpe bzw. der Schrittmotor der Dosierpumpe derart ausgeführt sein, dass der Start der Ansaugphase und/oder der Auslassphase einem Totpunkt der Dosierpumpe entspricht. Dadurch ist der Start der Ansaugphase und/oder der Auslassphase immer mit einer Kolbenposition bzw. einer Position des Schrittmotors synchronisiert und der Schrittmotor kann auf eine Positionsbestimmungseinheit wie beispielsweise einen Drehgeber verzichten.

Nach einem optionalen Aspekt der Offenbarung kann die Steuer- oder Regelungseinheit eine Drehzahl des Schrittmotors während der Ansaugphase der Dosierpumpe derart steuern oder regeln, dass eine Drehzahl des Schrittmotors während der Ansaugphase größer als eine Drehzahl des Schrittmotors während der Auslassphase der Dosierpumpe ist.

Der Schrittmotor der Dosierpumpe kann von der Steuer- oder Regelungseinheit dabei derart gesteuert oder geregelt werden, dass eine Ansaughub-Drehzahl und eine Auslasshub-Drehzahl ungleich sind. Die Ansteuerung der Dosierpumpe erfolgt also nicht mit einer kontinuierlichen Drehzahlvorgabe, sondern die Ansaughub-Drehzahl ist im Vergleich zu einer Auslasshub-Drehzahl beschleunigt. Somit ist die Ansaugphase kürzer als die Auslassphase. Das Additiv wird somit zumindest annähernd kontinuierlich von der Dosierpumpe abgegeben und die Fördercharakteristik der Dosierpumpe geglättet.

Es kann von Vorteil sein, wenn die Ansteuerungseinheit oder die Steuer- oder Regelungseinheit derart vorgesehen und ausgebildet ist, um die zu erzielende Reduzierung der Ansaugphase durch die maximal zu erreichende Drehzahl des Schrittmotors der Dosierpumpe oder der ersten Dosierpumpe und/ oder der zweiten Dosierpumpe zu begrenzen.

In anderen Worten ist es bevorzugt, wenn die vorstehende Vorrichtung eine Reduzierung der elektrischen Leitfähigkeitsschwankungen bei diskontinuierlicher Förderung mittels der Dosierpumpe durch unterschiedliche Antriebs(motor)-Geschwindigkeiten in der Ansaug- und Auslassphase durchführt.

Vorzugsweise kann eine Frequenz des Stroms, mit dem der Schrittmotor in der Auslassphase beaufschlagt wird, geringer sein als eine Frequenz des Stroms, mit dem der Schrittmotor in der Ansaugphase beaufschlagt wird. Dadurch kann die Drehzahl des Schrittmotors in der Auslassphase geringer sein als in der Ansaugphase.

Es ist vorteilhaft, wenn die Ansteuerungseinheit dazu vorgesehen und ausgebildet ist, um den Schrittmotor derart zu steuern, um zu Beginn und zum Ende der Auslassphase die Drehzahl der Dosierpumpe entsprechend einer Förderkennlinie der Dosierpumpe zu erhöhen, so dass über eine Mehrzahl von Ansaug-Auslasszyklen hinweg, eine annähernd konstante Förderung erzielbar ist. In anderen Worten werden zum Erreichen des vorstehenden Aspekts der Ansteuerung von der Ansteuerungseinheit während des Beginns der Auslassphase Schrittimpulse erzeugt, durch die die Rotationsgeschwindigkeit des Schrittmotors sequentiell von der ggf. maximal möglichen Drehzahl in der Ansaugphase verringert wird. Zum Ende der Auslassphase wird die Rotationsgeschwindigkeit wieder sequentiell auf die ggf. maximal mögliche Drehzahl für die Ansaugphase erhöht, so dass in der Auslassphase eine annähernd kontinuierliche Förderung entsteht.

Es ist ferner von Vorteil, wenn die Ansteuerungseinheit dazu ausgebildet und vorgesehen ist, um den Schrittmotor mit einer variierenden Drehzahl zu betreiben.

Die erste und die zweite Dosierpumpe ist vorzugsweise jeweils eine Drehschieberkolbenpumpe (DSK).

Es ist von Vorteil, wenn die Ansteuerungseinheit dazu vorgesehen und ausgebildet ist, um eine zeitliche Auflösung von Ansteuerimpulsen für den Schrittmotor zum Erzielen einer vorbestimmten Kompensation der Ansaugphase und/ oder Auslassphase zu berechnen.

Es ist von Vorteil, wenn die Ansteuerungseinheit ein Mikrocontroller ist, der vorzugsweise in einem Treiber für den Schrittmotor einer Dosierpumpe vorgesehen ist, oder die Ansteuerungseinheit in die Regelungseinheit integriert ist. In anderen Worten kann die Ansteuerung zum Beispiel durch einen Mikrocontroller in einem vorhandenen Treiber für einen Schrittmotor einer Dosierpumpe umgesetzt werden oder aber von einer bestehenden Recheneinheit der vorhandenen Regelungseinheit übernommen werden. Die von der Ansteuerungseinheit generierten Schrittimpulse werden an den Hardwaretreiber des Schrittmotors weitergeleitet, der dann eine Schrittbewegung des Schrittmotors auslöst.

Ferner betrifft die vorliegende Offenbarung ein Ansteuerungsverfahren einer Dosierpumpe zur Herstellung und/oder Aufbereitung von Dialysierflüssigkeit mit den folgenden Schritten:
- Erfassen eines Positionssignals eines Kolbens der Dosierpumpe durch einen magnetischen Sensor;
- Synchronisieren eines Schrittmotors zum Betreiben der Dosierpumpe mit einem Start der Ansaugphase und/oder der Auslassphase der Dosierpumpe, insbesondere durch das erfasste Positionssignal,
- Berechnung einer zeitlichen Auflösung von Steuerimpulsen des Schrittmotors durch die Steuer-oder Regelungseinheit, derart, dass der Schrittmotor in der Ansaugphase mit einer höheren Drehzahl dreht als in der Auslassphase, insbesondere, dass der der Schrittmotor in der Ansaugphase mit der maximalen Drehzahl dreht,
- Erzeugen der berechneten Steuerimpulse in einem erforderlichen Zeitraster.

In anderen Worten werden die Steuerimpulse des Schrittmotors als elektrischer Antrieb so berechnet, dass der Schrittmotor in der Ansaugphase schneller dreht als in der Auslassphase und somit die Ansaugphase kürzer als die Auslassphase ist.

Durch das offenbarungsgemäße Ansteuerungsverfahren kann eine Dosierpumpe in einer Vorrichtung gemäß einem der vorstehenden Aspekte angesteuert werden.

Die Steuer- oder Regelungseinheit gibt eine Stellgröße für den Schrittmotor der Dosierpumpe basierend auf der erfassten Ist-Leitfähigkeit aus. Diese ausgegebene Stellgröße ist sehr anfällig für die (regelmäßigen) Leitfähigkeitsschwankungen durch die unregelmäßige Fördermenge der Pumpe. Darum wird die Stellgröße der Steuer- oder Regelungseinheit derart verändert, dass die Steuerimpulse die Ansaugphase verkürzen und die Auslassphase verlängern. Dies kann entweder durch die Steuer- oder Regelungseinheit selbst oder durch die Ansteuereinheit geschehen.

Es ist bevorzugt, wenn das Ansteuerungsverfahren die folgenden Schritte aufweist:
- Erzeugen von Steuerimpulsen von der Ansteuerungseinheit während der Ansaugphase, die der maximal möglichen Drehzahl des Schrittmotors entsprechen,
- Erzeugen von Steuerimpulsen von der Ansteuerungseinheit während des Beginns der Auslassphase, durch die die Rotationsgeschwindigkeit des Schrittmotors sequentiell von der maximal möglichen Drehzahl in der Ansaugphase verringert wird,
- Erzeugen von Schrittimpulsen von der Ansteuerungseinheit zum Ende der Auslassphase, um die Rotationsgeschwindigkeit wieder sequentiell auf die maximal mögliche Drehzahl für die Ansaugphase zu erhöhen, so dass in der Auslassphase eine annähernd kontinuierliche Förderung entsteht.

Durch die Verringerung der Drehzahl bei Beginn der Auslassphase und Erhöhung der Drehzahl zum Ende der Auslassphase wird eine annähernd kontinuierliche Fördermenge/ein annähernd kontinuierlicher Volumenstrom über die gesamte Auslassphase erreicht.

Es von Vorteil, wenn die zu erzielende Reduzierung der Ansaugphase durch die maximal zu erreichende Drehzahl des Schrittmotors der Dosierpumpe begrenzt ist. Durch die Verwendung der maximalen Drehzahl in der Ansaugphase wird die Ansaugphase minimiert.

Es ist vorteilhaft, wenn die von der Ansteuerungseinheit generierten Steuerimpulse an einen Treiber, insbesondere einen Hardwaretreiber, des Schrittmotors weitergeleitet werden, die dann eine Schrittbewegung des Schrittmotors auslöst.

Die Aufgabe der vorliegenden Offenbarung wird zusätzlich durch ein computerlesbares Speichermedium und/oder ein Computerprogramm gelöst, die jeweils Befehle umfassen, die bei Ausführung durch einen Computer diesen veranlassen die Schritt des Ansteuerungsverfahrens gemäß den vorstehenden Aspekten auszuführen.

### Kurzbeschreibung der Figuren

Fig. 1 ist eine Darstellung zur Veranschaulichung eines Regelkreises gemäß der vorliegenden Offenbarung;
Fig. 2 ist eine schematische Darstellung zur Veranschaulichung eines Funktionsprinzips der Ansteuerungseinheit;
Figuren 3a bis 3d sind eine Darstellung zur Veranschaulichung des Funktionsprinzips einer Dosierpumpe;
Fig. 4 ist eine Darstellung zur Veranschaulichung einer Volumenverschiebung in einer Drehschieberkolbenpumpe;
Fig. 5a ist eine Darstellung zur Veranschaulichung des Verhaltens der Pumpenrotation bei konstanter Drehzahl;
Fig. 5b ist eine Darstellung zur Veranschaulichung des Verhaltens der Leitfähigkeit nach der Einspritzstelle;
Fig. 6a ist eine Darstellung zur Veranschaulichung des Verhaltens der Pumpenrotation bei einer Drehzahl gemäß der vorliegenden Offenbarung;
Fig. 6b ist eine Darstellung zur Veranschaulichung des Verhaltens der Leitfähigkeit nach der Einspritzstelle gemäß der vorliegenden Offenbarung;
Fig. 7a ist eine Darstellung zur Veranschaulichung des Verhaltens der Pumpenrotation bei einer Drehzahl gemäß der vorliegenden Offenbarung; und
Fig. 7b ist eine Darstellung zur Veranschaulichung des Verhaltens der Leitfähigkeit nach der Einspritzstelle gemäß der vorliegenden Offenbarung.

### Beschreibung der Ausführungsbeispiele

Nachstehend werden Ausführungsbeispiele der vorliegenden Offenbarung auf der Basis der zugehörigen Figuren beschrieben.

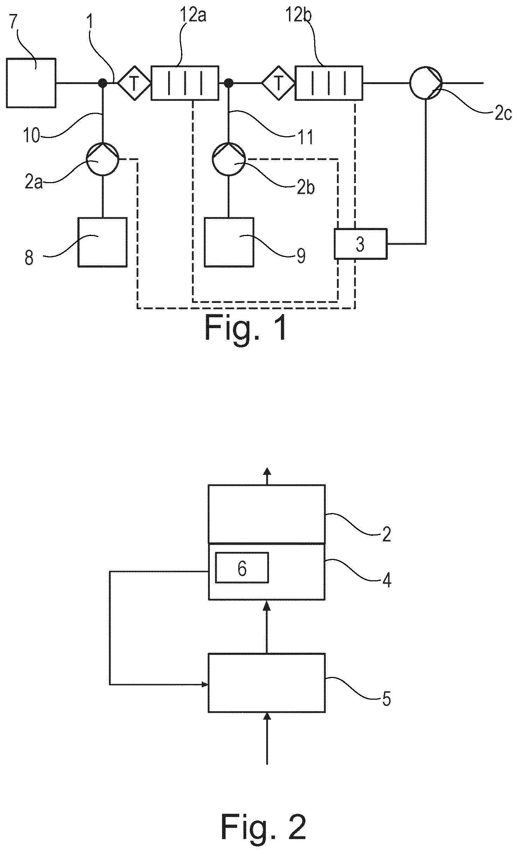

Fig. 1 ist eine Darstellung zur Veranschaulichung einer Vorrichtung/eines Regelkreises gemäß der vorliegenden Offenbarung. Die Vorrichtung bzw. der Regelkreis gemäß Fig. 1 weist eine Fluidhauptleitung 1 zur Führung einer Dialysierflüssigkeit auf. Die Fluidhauptleitung 1 beginnt in einer ersten Quelle 7, welche den Grundbestandteil (osmotisches) Wasser der Dialysierflüssigkeit bereitstellt. In einem ersten Vorrat 8 stellt die Vorrichtung/ der Regelkreis ein erstes Additiv bzw. eine erste Komponente, vorzugsweise ein basisches Fluid, zur Verfügung und in einem zweiten Vorrat 9 stellt die Vorrichtung/ der Regelkreis eine zweite Komponente/ein zweites Additiv, vorzugsweise ein saures Fluid, zur Verfügung.

Der erste Vorrat 8 ist über eine erste Regelstrecke/Fluidnebenleitung 10 mit der Fluidhauptleitung 1 verbunden. In der ersten Regelstrecke 10 ist eine erste Dosierpumpe 2a vorgesehen, welche dazu vorgesehen und ausgebildet ist, um die erste Komponente aus dem ersten Vorrat 8 in Richtung hin zu der Fluidhauptleitung 1 zu fördern und dieser zuzugeben.

Der zweite Vorrat 9 ist über eine zweite Regelstrecke/Fluidnebenleitung 11 mit der Fluidhauptleitung 1 verbunden. In der zweiten Regelstrecke 11 ist eine zweite Dosierpumpe 2b vorgesehen, welche dazu vorgesehen und ausgebildet ist, um die zweite Komponente aus dem zweiten Vorrat 9 in Richtung hin zu der Fluidhauptleitung 1 zu fördern und dieser zuzugeben.

Die Fluidhauptleitung 1 weist eine dritte Dosierpumpe 2c auf, die die gemischte Dialysierflüssigkeit zu einem Dialysator (nicht dargestellt) fördert.

In anderen Worten ist die Vorrichtung bzw. der Regelkreis derart vorgesehen und ausgebildet, dass aus der ersten Quelle 7 der Grundbestandteil (osmotisches) Wasser in die Fluidhauptleitung 1 gegeben wird, welches in einem ersten Schritt mit dem Fluid aus dem ersten Vorrat 8 vermischt wird und in einem weiteren Schritt mit dem Fluid aus dem zweiten Vorrat 9 vermischt wird.

Sowohl die erste Dosierpumpe 2a als auch die zweite Dosierpumpe 2b sind mit einer Steuer- oder Regelungseinheit 3 verbunden. In der Fluidhauptleitung 1 ist nach Zugabe der ersten Komponente dem ersten Vorrat 8 eine erste Sonde, vorzugsweise ein Temperatursensor T und eine Leitfähigkeitssonde 12a, angeordnet, welche ebenfalls mit der Steuer- oder Regelungseinheit 3 verbunden ist. Die Leitfähigkeitssonde 12a ist dazu vorgesehen und ausgebildet, um die Leitfähigkeit der mit dem osmotischen Wasser vermischten ersten (basischen) Komponente aus dem ersten Vorrat 8 nach deren Zugabe über die erste Regelstrecke 10 mittels der ersten Dosierpumpe 2a zu messen. Aus der erfassten Leitfähigkeit wird mithilfe der durch den Temperatursensor T erfassten Temperatur eine Konzentration der ersten Komponente in der Flüssigkeit ermittelt. Die Steuer- oder Regelungseinheit 3 ist dazu vorgesehen und ausgebildet, um den IST-Wert der Leitfähigkeit des Osmose-Wassers mit der ersten (basischen) Komponente mit dem Sollwert zu vergleichen und über eine Drehzahl der ersten Dosierpumpe 2a nach zu regeln, wenn notwendig.

In der Fluidhauptleitung 1 ist nach Zugabe der zweiten Komponente aus dem zweiten Vorrat 9 eine zweite Sonde, vorzugsweise ein Temperatursensor T und eine Leitfähigkeitssonde12b, angeordnet, welche ebenfalls mit der Steuer- oder Regelungseinheit 3 verbunden ist. Die zweite Sonde 12b ist dazu vorgesehen und ausgebildet, um die Leitfähigkeit der zweiten (sauren) Komponente aus dem zweiten Vorrat 9 nach deren Zugabe über die zweite Regelstrecke 11 mittels der zweiten Dosierpumpe 2b in Kombination mit der in der Fluidhauptleitung 1 bereits vorhandenen Flüssigkeit zu messen. In anderen Worten misst die zweite Sonde 12b die Leitfähigkeit der gesamten Dialysierflüssigkeit. Aus der erfassten Leitfähigkeit wird vorzugsweise mithilfe der durch den Temperatursensor T erfassten Temperatur die Konzentration der zweiten Komponente in der Dialysierflüssigkeit gemessen. Die Regelungseinheit 3 ist dazu vorgesehen und ausgebildet, um den IST-Wert der Leitfähigkeit der gesamten Dialysierflüssigkeit mit dem Sollwert zu vergleichen und über eine Drehzahl der zweiten Dosierpumpe 2b nach zu regeln, wenn notwendig.

Fig. 2 ist eine schematische Darstellung zur Veranschaulichung eines Funktionsprinzips einer Ansteuerungseinheit 5. Die Ansteuerungseinheit 5 ist entweder in die Steuer- oder Regelungseinheit 3 integriert bzw. wird von einer Rechnereinheit der Regelungseinheit 3 übernommen oder kann durch einen Mikrocontroller (nicht gezeigt) in einem vorhandenen Treiber für eine Dosierpumpe 2 umgesetzt werden. Fig. 2 ist hierbei eine schematische Darstellung oder ein Ablaufdiagramm, welches unabhängig davon, wie die Ansteuerungseinheit 5 umgesetzt wird, gilt.

Demnach hat die Dosierpumpe 2 einen Schrittmotor als eine Antriebseinheit / einen elektrischen Antrieb 4, welcher von der Ansteuerungseinheit 5 angesteuert wird. Die Ansteuerungseinheit 5 übersetzt gewissermaßen die Stellgröße der Steuer- oder Regelungseinheit 3 in einen Eingangswert für den elektrischen Antrieb 4, beispielsweise in den Steuerstrom, den Drehwinkel oder die Anzahl der Motorschritte des elektrischen Antriebs 4. Die Dosierpumpe 2 weist eine Positionsbestimmungseinheit 6 auf, die eine physikalische Position der Dosierpumpe 2 erfasst.

Die Positionsbestimmungseinheit 6 ist ein magnetischer Sensor. Die Positionsbestimmungseinheit 6 ist dazu vorgesehen und ausgebildet, um eine Synchronisation der physikalischen Position mit einem Start der Ansaugphase und/oder der Auslassphase durchzuführen. Der elektrischen Antrieb 4 wird also mit dem Start der Ansaugphase und/oder der Auslassphase, die durch die Positionsbestimmungseinheit 6 erfasst wird, synchronisiert. Die erkannte physikalische Position der Positionsbestimmungseinheit 6 wird der Ansteuerungseinheit 5 mitgeteilt.

Mittels der Ansteuerungseinheit 5 wird nach einer erstmaligen Synchronisation der physikalischen Position basierend auf einer Förderrate eine zeitliche Auflösung von Ansteuerimpulsen für den elektrischen Antrieb 4 zum Erzielen einer vorbestimmten Kompensation der Einlass-/Ansaugphase und/ oder Auslass-/Austragsphase berechnet. Die Ansteuerungseinheit 5 gibt Schrittimpulse an den elektrischen Antrieb 4 weiter, um die Drehzahl der Dosierpumpe 2 entsprechend zu erhöhen oder zu verringern.

Die Ansteuerungseinheit 5 steuert den elektrischen Antrieb 4 derart an, dass die erste Dosierpumpe 2a und/oder die zweite Dosierpumpe 2b mit einer beschleunigten Drehzahl während der Einlass-/Ansaugphase und einer reduzierten Drehzahl während der Auslassphase vorgesehen sind bzw. dass die Ansaugphase kürzer als die Auslassphase ist.

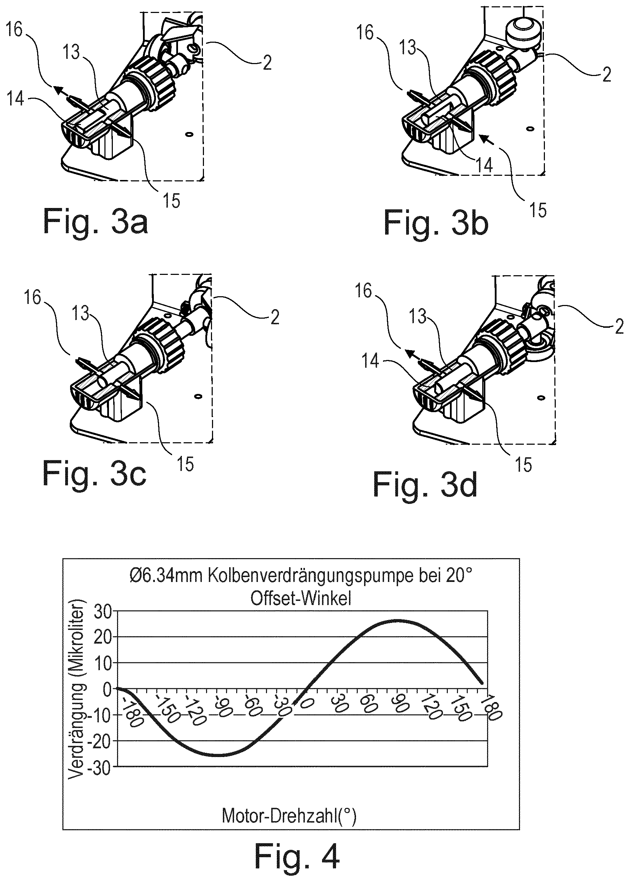

Figuren 3a bis 3d sind eine Darstellung zur Veranschaulichung des Funktionsprinzips einer Dosierpumpe 2, vorzugsweise einer Drehschieberkolbenpumpe, gemäß der vorliegenden Offenbarung. Hierbei beschreiben die Figuren 3a bis 3d vier zueinander unterschiedliche Positionen eines Kolbens 13 in einer solchen Drehschieberkolbenpumpe 2. Der Kolben 13 ist zylinderförmig und weist auf einer Seite eine Abflachung 14 auf. Die Abflachung 14 erstreckt sich über zumindest die Hälfte des in Fig. 3a sichtbaren vorderen Teils des Kolbens 13.

In einer ersten Position gemäß Fig. 3a befindet sich der Kolben 13 in einer oberen Totpunktlage, das heißt, die Abflachung 14 ist in Richtung nach oben gedreht/zeigend. In der oberen Totpunktlage ist die Dosierpumpe 2 dahingehend vorbereitet, um Fluid/Flüssigkeit einzulassen.

In einer zweiten Position gemäß Fig. 3b zieht sich der Kolben 13 zurück und zieht damit Fluid/Flüssigkeit über einen Einlass 15 in die Dosierpumpe 2. In der zweiten Position ist die Abflachung dem Einlass 15 zugewandt.

In einer dritten Position gemäß Fig. 3c befindet sich der Kolben 13 in einer unteren Totpunktlage, das heißt, die Abflachung 14 ist in Richtung nach unten gedreht/zeigend. In der unteren Totpunktlage ist die Dosierpumpe 2 vorbereitet, um Fluid/Flüssigkeit über einen Auslass 16 abzugeben.

In einer vierten Position gemäß Fig. 3d befindet sich der Kolben 13 in einer Position, in welcher der Kolben 13 Fluid/Flüssigkeit durch den Auslass 16 herausdrückt.

In anderen Worten bedeutet das, dass der Kolben 13 und somit die Abflachung 14 rotierend vorgesehen ist, sodass über die Höhe der Drehzahl die Rotationsgeschwindigkeit des Kolbens 13 steuerbar/ regelbar ist, um die Förderrate entsprechend beeinflussen zu können.

Fig. 4 ist eine Darstellung zur Veranschaulichung einer Volumenverschiebung oder eines Zylindervolumens in einer Drehschieberkolbenpumpe 2 mit einem (Kolben- )Durchmesser von 6,34 mm bei einem Offset-Winkel von 20°. Hierbei ist auf der x-Achse die Motor-Drehzahl bzw. ein Drehwinkel des elektrischen Antriebs 4 in Grad (°) angegeben und auf der y-Achse die Verdrängung in Mikroliter. Der gezeigte Verlauf entspricht einer allgemein bekannten Sinuskurve, welche bei ±180° und 0° jeweils einen Nulldurchgang aufweist.

Demnach ist die dargestellte Sinuskurve in vier Abschnitte unterteilbar. Der erste abfallende Kurvenabschnitt im Bereich von -180° bis -90° entspricht der vorstehend beschriebenen ersten Position. Der ansteigende Kurvenabschnitt im Bereich von -90° bis 0° entspricht der vorstehend beschriebenen zweiten Position. Der ansteigende Kurvenabschnitt im Bereich von 0° bis +90° entspricht der vorstehend beschriebenen dritten Position. Der abfallende Kurvenabschnitt im Bereich von +90° bis +180° entspricht der vorstehend beschriebenen vierten Position.

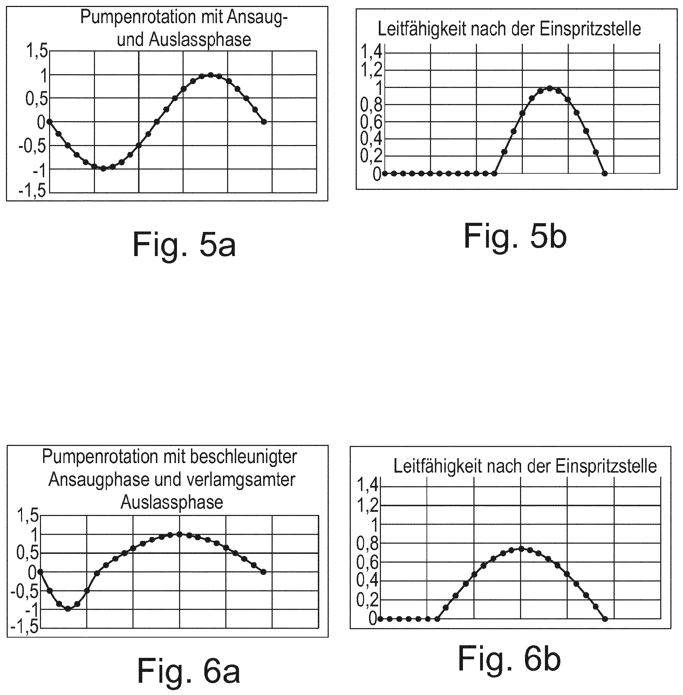

In den nachfolgend beschriebenen Diagrammen gemäß Figuren 5a, 5b, 6a, 6b, 7a und 7b ist jeweils einmal die Pumpenrotation über der Zeit und jeweils einmal die Leitfähigkeit über der Zeit dargestellt.

Fig. 5a ist eine Darstellung zur Veranschaulichung des Verhaltens der Pumpenrotation bei konstanter Drehzahl und Fig. 5b ist eine Darstellung zur Veranschaulichung des Verhaltens der Leitfähigkeit nach der Einspritzstelle in Abhängigkeit der Pumpenrotation gemäß Fig. 5a. Sowohl die x-Achse von Fig. 5a als auch die x-Achse von Fig. 5b beschreibt den Verlauf der Zeit.

In Fig. 5a bildet ein geförderter Volumenstrom der Pumpe über eine komplette Pumpenumdrehung eine allgemein bekannte Sinuskurve. Hierbei entspricht der negative Anteil des Volumenstroms der Ansaugphase und der positive Anteil der Auslass-/ Austragsphase. In Fig. 5b ist Leitfähigkeit nach der Einspritzstelle als Kennlinie dargestellt. Hierbei ist die Leitfähigkeit solange Null, wie die Sinuskurve der Pumpenrotation also der geförderte Volumenstrom der Pumpe Null oder negativ ist. Wenn das geförderte Volumen der Pumpe positiv wird, steigt die Leitfähigkeit an und zeigt eine nach unten geöffnete Parabel. Das Maximum der Leitfähigkeitskurve bei konstanter Drehzahl liegt bei 1.

Fig. 6a ist eine Darstellung zur Veranschaulichung des Verhaltens der Pumpenrotation bei einer Drehzahl gemäß der vorliegenden Offenbarung. Fig. 6a zeigt den geförderten Volumenstrom der Dosierpumpe 2 mit einer beschleunigten Drehzahl während der Ansaug-/Einlassphase und einer verlangsamten Drehzahl während der Auslass-/Austragsphase. Hierbei weist die Sinuskurve in/während der Einlassphase eine gestauchte Kurve und in/während der Auslassphase eine gestreckte Kurve auf. Dementsprechend verhält es sich mit dem Kurvenverlauf in Fig. 6b. D.h. der Bereich mit einer Leitfähigkeit ungleich Null ist größer. Dafür ist das Maximum der Leitfähigkeit über die Zeit geringer als in Fig. 5b.

Fig. 6b ist eine Darstellung zur Veranschaulichung des Verhaltens der Leitfähigkeit nach der Einspritzstelle gemäß der vorliegenden Offenbarung in Abhängigkeit von dem geförderten Volumenstrom gemäß Fig. 6a. Bei der Leitfähigkeits-Kennlinie der Fig. 6b in Abhängigkeit von der beschleunigten Ansaugphase und der verlangsamten Auslassphase ist der Verlauf der nach unten geöffneten Parabel abgeflacht im Gegensatz zu der Leitfähigkeitskurve bei konstanter Drehzahl. Demnach ist das Maximum der nach unten geöffneten Parabel der Leitfähigkeitskurve unterhalb von 0,8. Auf diese Weise kann eine kontinuierlichere Förderung von Fluid/Flüssigkeit ermöglicht werden.

In anderen Worten erfolgt gemäß Figuren 6a und 6b die Ansteuerung der Dosierpumpe 2 nicht mehr mit einer kontinuierlichen Drehzahlvorgabe, sondern mit beschleunigter Drehzahl während der Ansaugphase und reduzierter Drehzahl während der Auslassphase. Die während der Ansaugphase erzeugten Schrittimpulse von der Ansteuerungseinheit 5 entsprechen der maximal möglichen Drehzahl der Antriebseinheit 4.

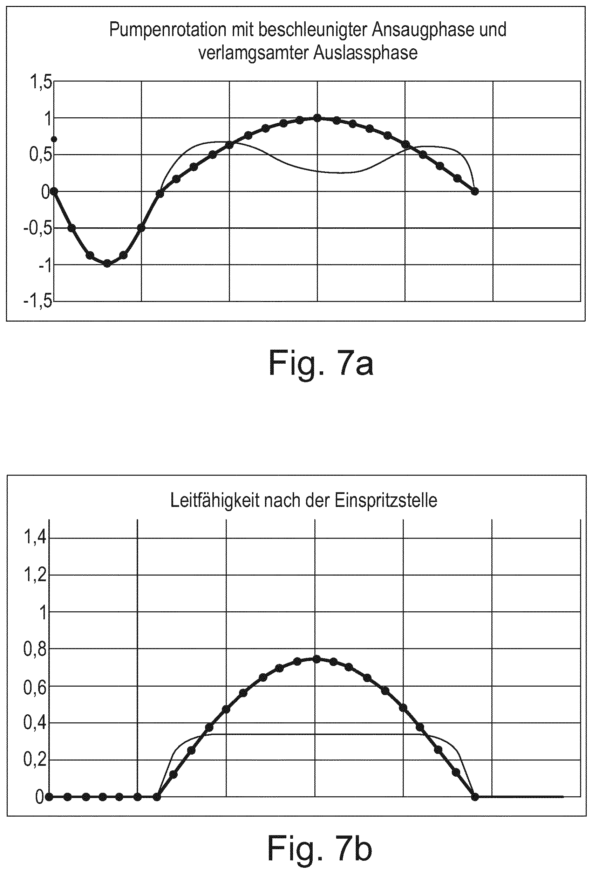

Fig. 7a ist eine Darstellung zur Veranschaulichung einer theoretischen Optimierung der Pumpenrotation bei einer variierenden Drehzahl und Fig. 7b ist eine Darstellung zur Veranschaulichung einer theoretischen Optimierung der Leitfähigkeit nach der Einspritzstelle gemäß der vorliegenden Offenbarung. Sowohl Fig. 7a als auch 7b zeigen den Kurvenverlauf der Figuren 6a und 6b. Im Vergleich hierzu ist jeweils eine weitere Kurve gezeigt, welche ohne einzelne Messpunkte und mittels eine dünneren Linie dargestellt ist.

In Fig. 7a ist ein Kurvenverlauf der Pumpenrotation gezeigt, bei welchem die Ansteuerungseinheit 5 während des Beginns der Auslassphase Schrittimpulse erzeugt, durch welche die Rotationsgeschwindigkeit der Antriebseinheit 4 sequentiell von der maximal möglichen Drehzahl in der Ansaugphase verringert wird. Zu Ende der Auslassphase wird die Rotationsgeschwindigkeit wieder sequentiell auf die maximal mögliche Drehzahl für die Ansaugphase erhöht, so dass in der Auslassphase eine annähernd kontinuierliche Förderung entsteht.

In anderen Worten wird der Kurvenverlauf der Pumpenrotation zu Beginn der Auslassphase nach oben kompensiert, anschließend wieder nach unten kompensiert, um gegen Ende der Auslassphase nochmals nach oben und wieder nach unten kompensiert zu werden. Auf dieser Weise wird der Kurvenabschnitt der Auslassphase abgeflacht.

Bei Betrachtung der Leitfähigkeitskurve gemäß Fig. 7b in Abhängigkeit von der Pumpenrotation gemäß Fig. 7a, ist zu erkennen, dass die nach unten geöffnete Parabel durch die vorstehend umgesetzten Drehzahlvariationen noch weiter abflachbar ist, sodass eine Art "Plateau" gemäß einer kontinuierlichen Förderung umsetzbar ist. Dieses "Plateau" hat einen Wert kleiner als 0,4 und größer 0,3.

### Bezugszeichen

- 1: Fluidhauptleitung
- 2: Dosierpumpe
- 3: Steuer- oder Regelungseinheit
- 4: elektrischer Antrieb (Schrittmotor)
- 5: Ansteuerungseinheit
- 6: Positionsbestimmungseinheit (magnetischer Sensor)
- 7: erste Quelle
- 8: erster Vorrat
- 9: zweiter Vorrat
- 10: erste Regelstrecke (erste Fluidnebenleitung)
- 11: zweite Regelstrecke (zweite Fluidnebenleitung)
- 12: Sonde
- 13: Kolben
- 14: Abflachung
- 15: Einlass
- 16: Auslass

## Patentansprüche

1. Vorrichtung für eine oder einer extrakorporalen Blutbehandlungsmaschine zur Herstellung und/oder Aufbereitung einer Dialysierflüssigkeit, mit
- einer Fluidhauptleitung (1) zur Einleitung von osmotischem Wasser,
- mindestens einer Fluidnebenleitung (10, 11), welche an die Fluidhauptleitung (1) für die Zufuhr eines Additivs in das osmotische Wasser angeschlossen ist,
- zumindest eine Dosierpumpe (2), die in einer Ansaugphase das Additiv aus mindestens einem Vorrat (8, 9) ansaugt und in einer seriell sich anschließenden Auslassphase das Additiv in das osmotische Wasser ausdrückt,
- einem elektrischen Antrieb zum Betreiben der Dosierpumpe (2) und
- einer Steuer- oder Regelungseinheit (3) zur Steuerung oder Regelung des elektrischen Antriebs, die den elektrischen Antrieb derart steuert oder regelt, dass die Ansaugphase kürzer ist als die Auslassphase,
**dadurch gekennzeichnet, dass**
der elektrische Antrieb ein Schrittmotor (4) zum Betreiben der Dosierpumpe (2) ist und die Dosierpumpe (2) einen magnetischen Sensor (6) aufweist, über den ein Positionssignal eines Kolbens (13) der Dosierpumpe (2) erfassbar ist, und die Steuer-oder Regelungseinheit (3) einen Start der Ansaugphase und/oder der Auslassphase mit dem erfassten Positionssignal synchronisiert.

2. Vorrichtung gemäß Anspruch 1, **dadurch gekennzeichnet, dass** der magnetische Sensor (6) das Positionssignal des Kolbens (13) an jeweiligen Totpunkten eines Kolbenhubs des Kolbens (13) der Dosierpumpe (2) erfasst.

3. Vorrichtung gemäß Anspruch 2, **dadurch gekennzeichnet, dass** die Steuer-oder Regelungseinheit (3) eine Position des Kolbens (13) ausgehend von dem erfassten Positionssignal anhand der von dem Schrittmotor (4) getätigten Schritte erfasst.

4. Vorrichtung gemäß einem der vorhergehenden Ansprüche 1 bis 3, **dadurch gekennzeichnet, dass** eine Drehzahl des Schrittmotors (4) während der Ansaugphase der Dosierpumpe (2) größer als eine Drehzahl des Schrittmotors (4) während der Auslassphase der Dosierpumpe (2) ist.

5. Vorrichtung gemäß Anspruch 4, **dadurch gekennzeichnet, dass** die Steuer-oder Regelungseinheit (3) dazu vorgesehen und ausgebildet ist, um den Schrittmotor (4) derart zu steuern, um zu Beginn und zum Ende der Auslassphase die Drehzahl Schrittmotors (4) der Dosierpumpe (2) entsprechend einer Förderkennlinie der Dosierpumpe (2) zu erhöhen, so dass eine annähernd konstante Förderung des Dosierpumpe (2) erzielbar ist.

6. Vorrichtung gemäß einem der vorhergehenden Ansprüche 1 bis 5, **dadurch gekennzeichnet, dass** der magnetische Sensor (6) ein Reedsensor oder ein Hallsensor ist.

7. Vorrichtung gemäß einem der vorhergehenden Ansprüche 1 bis 6, **dadurch gekennzeichnet, dass** die Steuer- oder Regelungseinheit (3) dazu vorgesehen und ausgebildet ist, um eine zeitliche Auflösung von Ansteuerimpulsen für den Schrittmotor (4) zum Erzielen einer vorbestimmten Kompensation der Ansaugphase und/ oder Auslassphase zu berechnen.

8. Vorrichtung gemäß einem der vorhergehenden Ansprüche 1 bis 7, **dadurch gekennzeichnet, dass** die Steuer- oder Regelungseinheit (3) ein Mikrocontroller ist, der vorzugsweise in einem Treiber für den Schrittmotor (4) einer Dosierpumpe (2) vorgesehen ist, oder eine Ansteuerungseinheit (5) in die Steuer- oder Regelungseinheit (3) integriert ist.

9. Ansteuerungsverfahren einer Dosierpumpe (2) zur Herstellung und/ oder Aufbereitung von Dialysierflüssigkeit mit den folgenden Schritten:
- Erfassen eines Positionssignals eines Kolbens (13) der Dosierpumpe (2) durch einen magnetischen Sensor (6);
- Synchronisieren eines Schrittmotors (4) zum Betreiben der Dosierpumpe (2) mit einem Start einer Ansaugphase und/oder einer Auslassphase der Dosierpumpe (2), insbesondere mit dem erfassten Positionssignal;
- Berechnung einer zeitlichen Auflösung von Steuerimpulsen des Schrittmotors (4) durch eine Ansteuerungseinheit (5), derart, dass der Schrittmotor (4) in der Ansaugphase mit einer höheren Drehzahl dreht als in der Auslassphase, insbesondere, dass der der Schrittmotor (4) in der Ansaugphase mit der maximalen Drehzahl dreht;
- Erzeugen der berechneten Steuerimpulse in einem erforderlichen Zeitraster.

10. Ansteuerungsverfahren gemäß Anspruch 9, **gekennzeichnet durch** die folgenden Schritte:
- Erzeugen von Steuerimpulsen von der Ansteuerungseinheit (5) während der Ansaugphase, die der maximal möglichen Drehzahl des Schrittmotors (4) entsprechen,
- Erzeugen von Steuerimpulsen von der Ansteuerungseinheit (5) während des Beginns der Auslassphase, durch die die Rotationsgeschwindigkeit des Schrittmotors (4) sequentiell von der maximal möglichen Drehzahl in der Ansaugphase verringert wird,
- Erzeugen von Schrittimpulsen von der Ansteuerungseinheit (5) zum Ende der Auslassphase, um die Rotationsgeschwindigkeit wieder sequentiell auf die maximal mögliche Drehzahl für die Ansaugphase zu erhöhen, so dass in der Auslassphase eine annähernd kontinuierliche Förderung entsteht.

11. Ansteuerungsverfahren gemäß Anspruch 10, **dadurch gekennzeichnet, dass** die zu erzielende Reduzierung der Ansaugphase durch die maximal zu erreichende Drehzahl des Schrittmotors (4) der Dosierpumpe begrenzt ist.

12. Ansteuerungsverfahren gemäß Anspruch 11, **dadurch gekennzeichnet, dass** die von der Ansteuerungseinheit (5) generierten Steuerimpulse an einen Hardwaretreiber der Antriebseinheit (4) weitergeleitet werden, die dann eine Schrittbewegung der Antriebseinheit (4) auslöst.

13. Computerlesbares Speichermedium, umfassend Befehle, die bei Ausführung durch die Steuer- oder Regelungseinheit der Vorrichtung nach einem der Ansprüche 1-8 diese veranlassen, die Schritte des Ansteuerungsverfahrens nach einem der Ansprüche 9 bis 12 auszuführen.

14. Computerprogramm, umfassend Befehle, die bei Ausführung durch die Steuer- oder Regelungseinheit der Vorrichtung nach einem der Ansprüche 1-8 diese veranlassen, die Schritte des Ansteuerungsverfahrens nach einem der Ansprüche 9 bis 12 auszuführen.

## Claims

1. A device for an or of an extracorporeal blood treatment machine for producing and/or processing a dialysis liquid, with
- a main fluid line (1) for introducing osmotic water,
- at least one secondary fluid line (10, 11) connected to the main fluid line (1) for supplying an additive into the osmotic water,
- at least one metering pump (2), which sucks in the additive from at least one storage means (8, 9) in an intake phase and discharges the additive into the osmotic water in a serially following discharge phase,
- an electric drive for operating the metering pump (2), and
- a control or regulation unit (3) for controlling or regulating the electric drive, which controls or regulates the electric drive in such a way that the intake phase is shorter than the discharge phase,
**characterized in that**
the electric drive is a stepper motor (4) for operating the metering pump (2) and the metering pump (2) has a magnetic sensor (6) via which a position signal of a piston (13) of the metering pump (2), can be detected, and the control or regulation unit (3) synchronizes a start of the intake phase and/or of the discharge phase with the detected position signal.

2. The device according to claim 1, **characterized in that** the magnetic sensor (6) detects the position signal of the piston (13) at respective dead centers of a piston stroke of the piston (13) of the metering pump (2).

3. The device according to claim 2, **characterized in that** the control or regulation unit (3) detects a position of the piston (13) starting from the detected position signal on the basis of the steps completed by the stepper motor (4).

4. The device according to one of the preceding claims 1 to 3, **characterized in that** a rotation speed of the stepper motor (4) during the intake phase of the metering pump (2) is greater than a rotation speed of the stepper motor (4) during the discharge phase of the metering pump (2).

5. The device according to claim 4, **characterized in that** the control or regulation unit (3) is provided and configured to control the stepper motor (4) in such a way as to increase the rotation speed of the stepper motor (4) of the metering pump (2) at the start and at the end of the discharge phase in accordance with a delivery characteristic of the metering pump (2), so that approximately constant conveying of the metering pump (2) can be achieved.

6. The device according to one of the preceding claims 1 to 5, **characterized in that** the magnetic sensor (6) is a reed sensor or a Hall sensor.

7. The device according to one of the preceding claims 1 to 6, **characterized in that** the control or regulation unit (3) is provided and configured to calculate a temporal resolution of drive pulses for the stepper motor (4) in order to achieve a predetermined compensation of the intake phase and/or discharge phase.

8. The device according to one of the preceding claims 1 to 7, **characterized in that** the control or regulation unit (3) is a microcontroller, which is preferably provided in a driver for the stepper motor (4) of a metering pump (2), or a driving unit (5) is integrated into the control or regulation unit (3).

9. A driving method of a metering pump (2) for producing and/or processing dialysis liquid comprising the following steps:
- detecting a position signal of a piston (13) of the metering pump (2) by a magnetic sensor (6);
- synchronizing a stepper motor (4) for operating the metering pump (2) with a start of an intake phase and/or of a discharge phase of the metering pump (2), in particular with the detected position signal;
- computing a temporal resolution of control pulses of the stepper motor (4) by a driving unit (5) such that the stepper motor (4) rotates at a higher rotation speed in the intake phase than in the discharge phase, in particular that the stepper motor (4) rotates at the maximum rotation speed in the intake phase;
- generating the calculated control impulses in a required time grid.

10. The driving method according to claim 9, **characterized by** the following steps:
- generating control pulses by the driving unit (5) during the intake phase that correspond to the maximum possible rotation speed of the stepper motor (4),
- generating control pulses by the driving unit (5) during the start of the discharge phase, by which the rotation velocity of the stepper motor (4) is sequentially reduced from the maximum possible rotation speed in the intake phase,
- generating step pulses by the driving unit (5) at the end of the discharge phase in order to sequentially increase the rotation velocity back to the maximum possible rotation speed for the intake phase, so that almost continuous conveying occurs in the discharge phase.

11. The driving method according to claim 10, **characterized in that** the reduction of the intake phase to be achieved is limited by the maximum rotation speed to be achieved by the stepper motor (4) of the metering pump.

12. The driving method according to claim 11, **characterized in that** the control pulses generated by the driving unit (5) are forwarded to a hardware driver of the drive unit (4), which then triggers a stepping movement of the drive unit (4).

13. A computer-readable storage medium comprising instructions which, when executed by the control or regulation unit of the device according to one of the claims 1-8, cause the control or regulation unit to perform the steps of the driving method according to one of claims 9 to 12.

14. The computer program comprising instructions which, when executed by the control or regulation unit of the device according to one of the claims 1-8, cause the control or regulation unit to perform the steps of the driving method according to one of claims 9 to 12.

## Revendications

1. Dispositif pour une machine de traitement extracorporel du sang destiné à la fabrication et/ou à la préparation d'un liquide de dialyse, avec
- une conduite principale de fluide (1) destinée à l'introduction d'eau osmotique,
- au moins une conduite auxiliaire de fluide (10, 11) qui est raccordée à la conduite principale de fluide (1) pour l'ajout d'un additif dans l'eau osmotique,
- au moins une pompe de dosage (2) qui aspire l'additif à partir d'au moins une réserve (8, 9) lors d'une phase d'aspiration et refoule l'additif dans l'eau osmotique lors d'une phase de refoulement qui s'ensuit en série,
- un entraînement électrique destiné au fonctionnement de la pompe de dosage (2) et
- une unité de commande ou de régulation (3) destinée à la commande ou la régulation de l'entraînement électrique qui commande ou régule l'entraînement électrique de telle sorte que la phase d'aspiration soit plus courte que la phase de refoulement,
**caractérisé en ce que**
l'entraînement électrique présente un moteur pas à pas (4) destiné au fonctionnement de la pompe de dosage (2), et la pompe de dosage (2) présente un capteur magnétique (6) par le biais duquel un signal de position d'un piston (13) de la pompe de dosage (2) peut être détecté, et l'unité de commande ou de régulation (3) synchronise un démarrage de la phase d'aspiration et/ou de la phase de refoulement avec le signal de position détecté.

2. Dispositif selon la revendication 1, **caractérisé en ce que** le capteur magnétique (6) détecte le signal de position du piston (13) au niveau de points morts respectifs d'une course de piston du piston (13) de la pompe de dosage (2).

3. Dispositif selon la revendication 2, **caractérisé en ce que** l'unité de commande ou de régulation (3) détecte une position du piston (13) à partir du signal de position détecté au moyen des pas effectués par le moteur pas à pas (4).

4. Dispositif selon l'une quelconque des revendications précédentes 1 à 3, **caractérisé en ce qu'**un régime du moteur pas à pas (4) pendant la phase de la phase d'aspiration de la pompe de dosage (2) est supérieure à un régime du moteur pas à pas (4) pendant la phase de refoulement de la pompe de dosage (2).

5. Dispositif selon la revendication 4, **caractérisé en ce que** l'unité de commande ou de régulation (3) est prévue et réalisée pour commander le moteur pas à pas (4) de manière à augmenter le régime du moteur pas à pas (4) de la pompe de dosage (2) au début et à la fin de la phase de refoulement conformément à une courbe de débit de la pompe de dosage (2) de telle sorte qu'un débit quasi constant de la pompe de dosage (2) puisse être obtenu.

6. Dispositif selon l'une quelconque des revendications précédentes 1 à 5, **caractérisé en ce que** le capteur magnétique (6) est un capteur reed ou un capteur à effet Hall.

7. Dispositif selon l'une quelconque des revendications précédentes 1 à 6, **caractérisé en ce que** l'unité de commande ou de régulation (3) est prévue et réalisée pour calculer une résolution temporelle d'impulsions de commande pour le moteur pas à pas (4) afin d'obtenir une compensation prédéterminée de la phase d'aspiration et/ou de la phase de refoulement.

8. Dispositif selon l'une quelconque des revendications précédentes 1 à 7, **caractérisé en ce que** l'unité de commande ou de régulation (3) est un microcontrôleur qui est prévu dans un pilote pour le moteur pas à pas (4) d'une pompe de dosage (2) ou une unité de commande (5) est intégrée dans l'unité de commande ou de régulation (3).

9. Procédé de commande d'une pompe de dosage (2) destiné à la fabrication et/ou à la préparation d'un liquide de dialyse avec les étapes suivantes :
- détection d'un signal de position d'un piston (13) de la pompe de dosage (2) par le biais d'un capteur magnétique (6) ;
- synchronisation d'un moteur pas à pas (4) destiné au fonctionnement d'une pompe de dosage (2) avec un démarrage d'une phase d'aspiration et/ou d'une phase de refoulement de la pompe de dosage (2), en particulier avec le signal de position détecté ;
- calcul d'une résolution temporelle d'impulsions de commande du moteur pas à pas (4) par le biais d'une unité de commande (5) de telle sorte que le moteur pas à pas (4) tourne lors de la phase d'aspiration avec un régime plus élevé que lors de la phase de refoulement, en particulier de telle sorte que le moteur pas à pas (4) tourne lors de la phase d'aspiration avec le régime maximal ;
- génération des impulsions de commande calculées selon une trame temporelle requise.

10. Procédé de commande selon la revendication 9, **caractérisé par** les étapes suivantes :
- génération d'impulsions de commande par l'unité de commande (5) pendant la phase d'aspiration qui correspondent au régime maximal possible du moteur pas à pas (4),
- génération d'impulsions de commande par l'unité de commande (5) pendant le début de la phase de refoulement par le biais de laquelle la vitesse de rotation du moteur pas à pas (4) est réduite séquentiellement par rapport au régime maximal possible pendant la phase d'aspiration,
- génération d'impulsions de pas par l'unité de commande (5) à la fin de la phase de refoulement pour augmenter à nouveau séquentiellement la vitesse de rotation jusqu'au régime maximal possible pour la phase d'aspiration de sorte que l'on obtient un débit quasi continu pendant la phase de refoulement.

11. Procédé de commande selon la revendication 10, **caractérisé en ce que** la réduction à obtenir de la phase d'aspiration est limitée par le régime maximal à atteindre du moteur pas à pas (4) de la pompe de dosage.

12. Procédé de commande selon la revendication 11, **caractérisé en ce que** les impulsions de commande générées par l'unité de commande (5) sont transmises à un pilote matériel de l'unité d'entraînement (4) qui déclenche alors un mouvement pas à pas de l'unité d'entraînement (4).

13. Support de stockage lisible par ordinateur, comprenant des instructions qui amènent, lors de la réalisation par l'unité de commande ou de régulation du dispositif selon l'une quelconque des revendications 1-8, celui-ci à réaliser les étapes du procédé de commande selon l'une quelconque des revendications 9 à 12.

14. Programme informatique, comprenant des instructions qui amènent, lors de la réalisation par l'unité de commande ou de régulation du dispositif selon l'une quelconque des revendications 1-8, celui-ci à réaliser les étapes du procédé de commande selon l'une quelconque des revendications 9 à 12.
